# EUROPEAN PATENT APPLICATION

(11) **EP 4 768 079 A2**
(43) Date of publication of application: **01.07.2026**
(21) Application number: 26164017.1
(22) Date of filing: 25.05.2022
(51) Int. Cl.: A61P 19/10

(54) **TREATMENT AND PREVENTION OF OSTEOPOROSIS IN HIGH BODY MASS INDEX INDIVIDUALS**

(30) Priority: 25.05.2021 US 202163192935 P
(62) Divisional of application: 22731804.5
(71) Applicant: Radius Health, Inc., Boston MA 02210 (US)
(72) Inventor: NIKONOVA, Elena, Boston, 02210 (US); WANG, Yamei, Boston, 02210 (US)
(74) Representative: Carpmaels & Ransford LLP

(57) **Abstract**

Disclosed herein is a method for preventing or reducing bone fractures in an overweight subject. The method comprises administering to the subject a therapeutically effective amount of an osteoanabolic agent.

## Description

### RELATED APPLICATIONS

The present application claims priority to US Provisional Application No. 63/192,935, filed May 25, 2021, which is incorporated herein by this reference in its entirety, including drawings.

### SUMMARY OF THE INVENTION

Low bone mineral density (BMD) is a major risk factor for osteoporosis and its related fractures. The relationship between body mass index (BMI), weight, height, and BMD has been studied for women (Kofi et al. "The Association between Body Mass Index and Osteoporosis in Patients Referred for a Bone Mineral Density Examination", Journal of Women's Health, Nov 2006, 1028-1034). Body weight or BMI has been found to be inversely related to bone mineral density. Compared to subjects having a moderate BMI, subjects with low BMI show a significantly lower BMD and are therefore at a higher risk of developing osteoporotic related fractures. Low BMI is an established risk factor for osteoporotic fracture; however, in recent years it has become evident that both low BMI and high BMI are associated with increased fracture risk depending on the skeletal site. In addition, obese patients may have higher fracture risk after adjustment for bone mineral density. Finally, the risk of fracture increases after weight loss in individuals that have a high BMI.

It has been found that, contrary to what is commonly understood, the use of osteoanabolic agents in high BMI subjects has a prophylactic effect on the incidence of bone fractures in individuals having a high BMI. It was further found that subjects that have, or had, a high BMI, and have lost a significant amount of weight (at least more than 5 kg) are more susceptible to bone fractures. Treatment with osteoanabolic agents can also be highly effective at reducing the incident of bone fractures in subjects that have lost significant amounts of weight due to dieting or bariatric surgery. Treatment of subjects with osteoanabolic agents prior to bariatric surgery and/or after bariatric surgery, can reduce the incidence of weight loss induced bone fractures in such patients.

Weight loss, particularly rapid weight loss induced, for example, by dieting or bariatric surgery, is generally associated with a decrease in BMD. It is believed that the proactive treatment of subjects that are undergoing rapid weight loss can inhibit, or prevent, the decrease in BMD experienced by such subjects. Inhibition of the loss of BMD contributes to the prevention of bone fractures in subjects that experience rapid weight loss.

Provided herein are methods for preventing or reducing bone fractures in a subject having a body mass index (BMI) greater than or equal to 25. The method comprises administering to the subject a therapeutically effective amount of an osteoanabolic agent.

Provided herein are methods for improving bone mineral density (BMD) and/or trabecular bone score (TBS) in a bone in a subject having a body mass index (BMI) greater than or equal to 25. The method comprises administering to the subject a therapeutically effective amount of an osteoanabolic agent.

In an embodiment, the administration of an osteoanabolic agent is initiated when the subject is at an age of at least about 40 years. In an embodiment, the administration of an osteoanabolic agent is initiated when the subject has a bone mineral density (BMD) that is below normal.

Provided herein are methods of reducing the incidence of bone fractures in a subject having a body mass index (BMI) greater than or equal to 25 when the subject is losing weight at a rate of at least 1 kg/month. The method comprises administering to the subject a therapeutically effective amount of an osteoanabolic agent.

Provided herein are methods of reducing the incidence of bone fractures in a subject after the subject has undergone bariatric surgery. The method comprises administering to the subject a therapeutically effective amount of an osteoanabolic agent. In an embodiment, the subject has a body mass index (BMI) greater than or equal to 25 prior to the bariatric surgery.

In an embodiment, the osteoanabolic agent is abaloparatide. Abaloparatide, in some embodiments, may be administered by subcutaneous injection.

In an embodiment, administration of a therapeutically effective amount of an osteoanabolic agent to the subject maintains or increases the subject's bone mineral density (BMD) during weight loss by the patient.

In some embodiments, the method further comprises administering to the subject a therapeutically effective amount of an anti-resorptive agent.

In an embodiment of the method, the subject is a woman. Women treated according to the method may be menopausal. The subject, in some embodiments, has osteopenia. In other embodiments, the subject has a normal BMD prior to treatment with the osteoanabolic agent.

The methods described herein result in a BMD increase of at least about 3% at one or more sites selected from the group consisting of spine, hip, and wrist.

### BRIEF DESCRIPTION OF DRAWINGS

FIG. 1 depicts a bar graph comparing the incidence of new vertebral fractures in overweight subjects treated with abaloparatide, teriparatide, or placebo.
FIG. 2 depicts bar graphs comparing the Kaplan-Meier estimates of fracture for non-vertebral fractures (NVF), clinical fractures (Clinical), major osteoporotic fractures (MOF) and wrist fractures (Wrist) in overweight subjects treated with abaloparatide, teriparatide, or placebo.
FIG.3 depicts line graphs comparing the bone mass density (BMD) percent change from baseline over time for total hip, femoral neck, and lumbar spine in overweight subjects treated with abaloparatide, teriparatide, or placebo.

### DETAILED DESCRIPTION OF THE INVENTION

The following description of the invention is merely intended to illustrate various embodiments of the invention. As such, the specific modifications discussed are not to be construed as limitations on the scope of the invention. It will be apparent to one skilled in the art that various equivalents, changes, and modifications may be made without departing from the scope of the invention, and it is understood that such equivalent embodiments are to be included herein.

Parathyroid hormone-related protein (PTHrP) refers to native human PTHrP (hPTHrP) and fragments thereof. PTHrP is a protein with homology to parathyroid hormone (PTH) at the amino-terminus that binds to the same G-protein coupled receptor. Despite a common receptor, parathyroid hormone receptor (PTHR), PTH primarily acts as an endocrine regulator of calcium homeostasis, whereas PTHrP plays a fundamental paracrine role in the mediation of endochondral bone development. The differential effects of these proteins may be related not only to differential tissue expression, but also to distinct receptor binding properties. Over the past several years, both PTHrP and PTH have been investigated as a potential treatment for osteoporosis. The results of these studies have been mixed, with some suggesting that intermittent administration of high dose PTHrP increases bone formation without concomitant stimulation of bone resorption and others reporting measurable stimulation of bone resorption and significant hypercalcemia (15-17).

A "fragment" of PTHrP refers to a polypeptide having a sequence comprising less than the full complement of amino acids found in PTHrP, which nonetheless elicits a similar biological response. Typically, fragments for use in the methods and compositions provided herein will be truncated from the C-terminus and will range from 30 to 40 residues in length. In particular, PTHrP(1-34), as well as analogues thereof with between 1 and 15 substitutions, are useful in the methods and compositions of the present invention.

As used herein, an "analogue" of PTHrP refers to a polypeptide having between about 1 and about 20, between about 1 and about 15, or between about 1 and about 10 art-accepted substitutions, additions, or insertions relative to PTHrP (i.e., relative to hPTHrP or a fragment thereof), or combinations thereof, not to exceed a total combination of 20 substitutions, additions, and insertions. As used herein, "insertions" include the insertion of an amino acid between two existing amino acids in the peptide chain. As used herein, "addition" means the addition of an amino acid to the N or C terminus of the peptide chain. As used herein, "substitution" means the substitution of an amino acid for an existing amino acid in the peptide chain. As used herein, "art-accepted" substitutions, insertions, or additions are those which one of ordinary skill in the art would expect to maintain or increase the biological and/or hormonal activity of the peptide and not adversely affect the biological activity of the peptide. Art-accepted substitutions include, for example, substitution of one amino acid with a chemically or biologically similar amino acid, such as substituting one hydrophobic amino acid for another hydrophobic amino acid. PTHrP analogues are described with reference to their variation from the native sequence of hPTHrP.

The term "osteoanabolic" refers to any therapy that promotes an increase in bone mass. At this time there are two osteoanabolic agents that are approved for the treatment of osteoporosis in the United States. Teriparatide is an osteoanabolic agent that is a PTH analogue. Abaloparatide is an osteoanabolic agent that is a PTHrP analogue.

Abaloparatide offers some advantages over teriparatide. Abaloparatide has potent anabolic activity with decreased bone resorption, less calcium-mobilizing potential, and improved room temperature stability. Studies performed in animals have demonstrated marked bone anabolic activity for the PTHrP analogue abaloparatide, with complete reversal of bone loss in ovariectomy-induced osteopenic rats and monkeys.

One subset of the general population that is particularly susceptible to osteoporotic fractures are people having high body mass index (BMI). BMI is calculated by taking a subject's weight, in kilograms, and dividing the weight by the square of the subject's height in meters. The resulting "index" number can be used to qualitatively categorize the weight of the subject as "underweight", "normal", "overweight" or "obese". A subject having a BMI of below about 18.5 is considered underweight. A subject having a BMI between about 18.5 and about 24.9 is considered normal (or healthy) weight. A subject having a BMI at or above 25 is considered overweight. A subject having a BMI at or above 30 is considered obese. As used herein the phrase "overweight subject" is defined as a person having a BMI greater than or equal to 25.

Generally, subjects that have a BMI at or above 25 (i.e., an overweight subject) have a higher BMD than a subject having a BMI below 25. This is because, in part, the body of an overweight subject compensates for the additional weight by increasing the bone density. For this reason, it is thought that overweight subjects are less likely to develop osteoporosis, or suffer osteoporotic related fractures. However, the osteoprotective effect of being overweight has been found to be overtaken by age related osteoporosis and estrogen deficiencies, causing overweight subjects to develop osteoporosis, although potentially at a later time then a normal weight subject.

In an embodiment of the invention, bone fractures can be prevented or reduced in a subject having a body mass index (BMI) greater than or equal to 25 by administering to the subject a therapeutically effective amount of an osteoanabolic agent. Administration of an osteoanabolic agent may help prevent BMD from decreasing. For example, as the subject ages bone resorption will begin to outpace bone formation. This occurs typically around the age of 50. In subjects that have a BMI greater than or equal to 25, this age related bone density loss may be masked or delayed due to the increase in BMD brought on by the higher than normal weight of the subject. Osteoporosis in such overweight subject can be inhibited or prevented by administration of osteoanabolic agents prior to the loss of BMD. This proactive treatment may be implemented by initiating treatment in overweight subjects when they reach the age of 40 years, 45 years, 50 years, 52 years, 54 years, 56 years, 58 years, or 60 years. Such treatment can delay or prevent osteoporosis.

Osteoporosis and osteopenia and typically determined by comparing a subject's bone mineral density test to the BMD of a healthy young adult (BMD_{Ref}). This is sometimes known as a "BMD T-Score". A BMD in a subject that is within 1 standard deviation (SD) of BMD_{Ref} (BMD T-Score of -1) is considered "normal". A BMD in a subject that is between 1 and 2.5 SD below BMD_{Ref} (BMD T-Score between -1 and -2.5) is considered "low" and such subjects are considered to exhibit "osteopenia." A BMD in a subject that is greater than 2.5 SD below BMD_{Ref} (BMD T-Score greater than -2.5) is considered to exhibit "osteoporosis". In an embodiment, treatment of overweight subjects may be started when osteopenia is found in the subject, (i.e., when the BMD is below "normal").

In another embodiment, a proactive treatment with osteoanabolic agents can be initiated when a female subject, that is overweight, begins menopause or is about to begin menopause. At menopause, lower levels of estrogen lead to a reduction in bone growth. Initiation of treatment with osteoanabolic agents may delay or prevent the accelerated loss in BMD seen in overweight, menopausal, subjects. For female subjects, proactive treatments for menopausal induced osteoporosis should begin by the age of 40 years, 42 years, 44 years, 48, years, 50 years, 52 years, 54 years, 56 years, 58 years, or 60 years.

Increases in weight of a subject can increase bone mass and alter bone architecture by inducing an adaptive response to the increased stress on the bones. Changes in weight therefore can cause increased BMD. This process is reversed when the weight is removed from the bones of the subject. Thus, increases in weight cause the bones to adjust by becoming denser, and loss of weight causes the bones to adjust be becoming less dense. (Iwaniec et al. "Influence of body weight on bone mass, architecture and turnover" J. of Endrocrinology (2016) 230, R115-R130). When a subject is undergoing regular or rapid weight loss, the bones of the subject will adjust to the reduced weight by becoming less dense. In an embodiment, a subject that is losing weight at a rate of at least 1 kg/month may be treated with an osteoanabolic agent. The proactive treatment of the subject during weight loss may inhibit or prevent the expected loss in BMD that accompanies such weight loss, which, in turn, should reduce the incidence of bone fractures in a subject. In an embodiment, the subject losing weight at a rate of at least 1 kg/month is an overweight subject, or a subject that was overweight prior to the beginning of the weight loss, as characterized by BMI.

Bariatric surgery, as used herein, refers to any surgery that makes a change to the digestive system with the goal of helping the subject lose weight. The most common bariatric surgeries include, but are not limited to, gastric bypass; bilopancreatic diversion with duodenal switch (BPD/DS); and sleeve gastrectomy. Bariatric surgery is typically performed in subjects that have a BMI of greater than or equal to 25. More often, the subject receiving bariatric surgery has a BMI of greater than 30, greater than 35 or greater than 40. After bariatric surgery is completed, the subject will experience rapid weight loss (weight loss of at least about 1 kg/month). As discussed previously, such rapid weight loss can lead to a loss of BMD. In an embodiment, a subject that has undergone bariatric surgery may be treated with an osteoanabolic agent to inhibit or prevent loss of BMD. The proactive treatment of the subject after bariatric surgery may inhibit or prevent the expected loss in BMD that accompanies rapid weight loss, which, in turn, should reduce the incidence of bone fractures in a subject. In an embodiment, the subject undergoing bariatric surgery was overweight prior to the surgery, as characterized by BMI.

In one embodiment, the osteoanabolic agent is abaloparatide. Overweight subjects treated with abaloparatide exhibited a significant reduction in certain bone fractures as compared to subjects treated with a placebo. When compared to subjects treated with placebo, overweight subjects treated with abaloparatide unexpectedly showed a statistically significant reduction in major osteoporotic fractures, clinical fractures, new vertebral fractures, and non-vertebral fractures in an 18-month trial (see Examples).

Subjects treated with abaloparatide also unexpectedly show a significant reduction in the risk of non-vertebral fractures (e.g., wrist fractures), and clinical fractures. Abaloparatide was further found to significantly decrease the risk of major osteoporotic fracture and any clinical fracture in postmenopausal overweight women, irrespective of baseline fracture probability, using the Fracture Risk Assessment Tool (FRAX).

Subjects treated with abaloparatide exhibit a significant increase not only in BMD, but also in trabecular bone score (TBS). TBS is a grey-scale textural analysis applied to spinal dual X-ray absorptivity (DXA) images that has been shown to be correlated with trabecular bone microarchitecture and bone strength. TBS is also a predictor of fragility fractures of the spine and hip in postmenopausal overweight women independent of BMD and other major clinical risk factors. As such, it captures additional patients at risk of fracture that are missed by BMD alone, and together with BMD more accurately captures bone strength.

Although a lower BMD is usually associated with higher fracture risk, a normal or even slightly higher than normal BMD does not necessarily indicate a lower fracture risk. For example, subjects that are overweight, as characterized by having a BMI greater than or equal to 25, may have increased fracture risk despite exhibiting a higher BMD.

Subjects treated with abaloparatide for 18 months unexpectedly demonstrated significant BMD increase in total hip and femoral neck versus subjects treated with teriparatide or placebo. Abaloparatide demonstrated a statistically significant increase in lumbar spine BMD at 6 months and 12 months versus placebo, and a non-statistically significant BMD increase at 18 months. Without wishing to be bound by any theory, an earlier increase in bone formation marker P1NP in subjects treated with abaloparatide may contribute to the faster effects of abaloparatide on BMD. For the CTX marker (bone resorption), subjects treated with abaloparatide showed a quick return to the baseline at 18 months.

Furthermore, subjects treated with abaloparatide for 18 months followed by an anti-resorptive therapy (e.g., alendronate for 6 months) showed a significant reduction in fracture risk versus subjects treated with placebo for 18 months followed by similar anti-resorptive therapy.

One aspect of the present disclosure relates to a method for preventing or reducing bone fractures in an overweight subject in need thereof comprising administering to the overweight subject a therapeutically effective amount of an osteoanabolic agent (e.g., abaloparatide). Exemplary bone fractures which may exhibit reduced fracture risk include, but are not limited to, major osteoporotic fractures (e.g., high- or low- trauma clinical fractures of the clinical spine, forearm, hip, or shoulder), non-vertebral fractures (e.g., wrist, hips, etc.), clinical fractures (e.g., fractures with or without high trauma, confirmed through x-ray scan, radiologist report, emergency room/urgent care reports, hospital discharge reports, surgery reports, hospital or clinical notes, or other medical confirmation), and new vertebral fractures.

Another aspect of the present disclosure relates to a method for preventing or reducing non-vertebral bone fractures in an overweight subject in need thereof comprising administering to the subject a therapeutically effective amount of an osteoanabolic agent (e.g., abaloparatide).

Another aspect of the present disclosure relates to a method for preventing or reducing vertebral bone fractures in an overweight subject in need thereof comprising administering to the subject a therapeutically effective amount of an osteoanabolic agent (e.g., abaloparatide).

Also provided herein is a method for preventing or reducing bone fractures in a subject that is undergoing rapid weight loss comprising administering to the subject a therapeutically effective amount of an osteoanabolic agent (e.g., abaloparatide).

Also provided herein is a method for preventing or reducing bone fractures in a subject after the subject has undergone bariatric surgery comprising administering to the subject a therapeutically effective amount of an osteoanabolic agent (e.g., abaloparatide) for a period of time.

Another aspect of the present disclosure relates to a method for improving or maintaining BMD and/or TBS in an overweight subject in need thereof comprising administering to the subject a therapeutically effective amount of an osteoanabolic agent (e.g., abaloparatide). Examples of bones which may exhibit improved BMD and/or TBS following administration include, without limitation, the lumbar spine, total hip, wrist, femur, cortical bone of the femur (femoral diaphysis), and/or femoral neck in the subject.

In certain embodiments, the therapeutic methods provided herein further comprise administering an anti-resorptive therapy following treatment with an osteoanabolic agent (e.g., abaloparatide) for an extended period of time. For example, provided herein is a method for improving BMD and/or trabecular bone score (TBS) in an overweight subject comprising administering to the subject a therapeutically effective amount of an osteoanabolic agent (e.g., abaloparatide) for a period of time, and subsequently administering to the subject a therapeutically effective amount of an anti-resorptive agent. Examples of bones which may exhibit improved BMD and/or TBS following administration include, without limitation, the lumbar spine, total hip, wrist, femur, cortical bone of the femur (femoral diaphysis), and/or femoral neck in the subject.

Also provided herein is a method for preventing or reducing bone fractures in a subject that is undergoing rapid weight loss comprising administering to the subject a therapeutically effective amount of an osteoanabolic agent (e.g., abaloparatide) for a period of time, and subsequently administering to the subject a therapeutically effective amount of an anti-resorptive agent. Exemplary bone fractures that may exhibit reduced fracture risk include, without limitation, major osteoporotic fracture, non-vertebral fracture (e.g., wrist, hip), clinical fracture, and new vertebral fracture. In those methods provided herein that comprise administration of an osteoanabolic agent followed by administration of an anti-resorptive agent, administration of the osteoanabolic agent analogue and anti-resorptive agent may overlap for some period of time, i.e., administration of the anti-resorptive agent may be initiated while the subject is still receiving the osteoanabolic agent.

Also provided herein is a method for preventing or reducing bone fractures in a subject after the subject has undergone bariatric surgery comprising administering to the subject a therapeutically effective amount of an osteoanabolic agent (e.g., abaloparatide) for a period of time, and subsequently administering to the subject a therapeutically effective amount of an anti-resorptive agent. Exemplary bone fractures that may exhibit reduced fracture risk include, without limitation, major osteoporotic fracture, non-vertebral fracture (e.g., wrist, hip), clinical fracture, and new vertebral fracture. In those methods provided herein that comprise administration of an osteoanabolic agent followed by administration of an anti-resorptive agent, administration of the osteoanabolic agent analogue and anti-resorptive agent may overlap for some period of time, i.e., administration of the anti-resorptive agent may be initiated while the subject is still receiving the osteoanabolic agent.

It is within the purview of one skilled in the art to select a suitable anti-resorptive therapy for the aspects and embodiments disclosed in this application. In some embodiments, the anti-resorptive therapeutic agents include bisphosphonates, estrogens, selective estrogen receptor modulators (SERMs), calcitonin, cathepsin K inhibitors, and monoclonal antibodies such as denosumab. In certain embodiments, the anti-resorptive therapeutic agent may be a bisphosphonate such as alendronate.

The term "subject in need thereof" as used herein refers to a mammalian subject, e.g., a human. In certain embodiments, a subject in need thereof has a fracture risk higher than normal. In certain embodiments, a subject in need thereof has one or more conditions selected from the group consisting of low BMD and high cortical porosity. BMD may be measured by digital X-ray radiogrammetry (DXR) or other methods known in the art. As used herein, the term "cortical porosity" means the fraction of cortical bone volume that is not occupied by the bone. Cortical porosity may be measured by DXR or other methods known in the art to provide an estimation of the local intensity minima ("holes") in the cortical bone regions using a recursive (climbing) algorithm starting from the outer region. A combined porosity measure is derived from the area percentage of holes found in the cortical part relative to the entire cortical area, by averaging over the involved bones and scaling to reflect a volumetric ratio rather than the projected area. A "high cortical porosity" means a porosity of about 10% higher, about 15% higher, about 20% higher, about 50% higher, about 100% higher, or about 150% higher than that of healthy subjects from the same age group as controls. For example, the subject may have a cortical porosity of about 0.01256, which the control group has a cortical porosity of about 0.01093. Subjects having a high cortical porosity may have a slightly low BMD, a normal BMD, or even a slightly higher than normal BMD, e.g., a BMD T-score of at least about -2, at least about -1.5, at least about -1, at least about -0.5, at least about -0.25, at least about -0.2, at least about -0.1, at least about 0, about -2 to about 3, about -2 to about 2.5, about -2 to about 2, about -2 to about 1.5, about -2 to about 1, about -2 to about 0.5, about -2 to about 0.25, about -2 to about 0.2, about -2 to about 0.1, or about -2 to about 0. For example, subjects with type II diabetes may have a cortical porosity up to twice that of controls while having normal or even slightly higher than normal BMD. Examples of suitable overweight subjects in need thereof include, without limitation, women, women with osteoporosis and/or diabetes (e.g., type I or type II diabetes), postmenopausal women, postmenopausal women with osteoporosis and/or diabetes (e.g., type I or type II diabetes), and men with osteoporosis and/or diabetes (e.g., type I or type II diabetes).

The term "therapeutically effective amount" as used herein refers to an amount of a compound or agent that is sufficient to elicit the required or desired therapeutic and/or prophylactic response, as the particular treatment context may require. In certain embodiments, the therapeutically effective amount is an amount of the composition that yields maximum therapeutic effect. In other embodiments, the therapeutically effective amount yields a therapeutic effect that is less than the maximum therapeutic effect. For example, a therapeutically effective amount may be an amount that produces a therapeutic effect while avoiding one or more side effects associated with a dosage that yields maximum therapeutic effect. A therapeutically effective amount for a particular composition will vary based on a variety of factors, including but not limited to the characteristics of the therapeutic composition (e.g., activity, pharmacokinetics, pharmacodynamics, and bioavailability), the physiological condition of the subject (e.g., age, body weight, sex, disease type and stage, medical history, general physical condition, responsiveness to a given dosage, and other present medications), the nature of any pharmaceutically acceptable carriers in the composition, and the route of administration. One skilled in the clinical and pharmacological arts will be able to determine a therapeutically effective amount through routine experimentation, namely by monitoring a subject's response to administration of a composition and adjusting the dosage accordingly. For additional guidance, see, e.g., Remington: The Science and Practice of Pharmacy, 22nd Edition, Pharmaceutical Press, London, 2012, and Goodman & Gilman's The Pharmacological Basis of Therapeutics, 12th Edition, McGraw-Hill, New York, NY, 2011, the entire disclosures of which are incorporated by reference herein.

Examples of therapeutically effective amounts of abaloparatide include, without limitation, about 10 µg to about 250 µg, about 50 µg to about 200 µg, about 50 µg to about 150 µg, about 70 µg to about 100 µg, about 70 µg to about 90 µg, about 75 µg to about 85 µg, about 20 µg, about 40 µg, about 60 µg, about 80 µg, about 100 µg, about 120 µg, about 150 µg, about 200 µg, or about 250 µg. Other examples of therapeutically effective amounts of abaloparatide may also include, without limitation, about 5 µg/kg or about 20 µg/kg. Depending on the particular anti-resorptive agent, one skilled in the art can select a therapeutically effective amount of the anti-resorptive agent. The amount of the anti-resorptive agent can be further optimized when used in combination with or subsequent to the therapy with abaloparatide.

In certain embodiments, osteoanabolic agents (e.g., abaloparatide) are administered by subcutaneous injection or transdermal administration.

In certain embodiments, osteoanabolic agents are administered for a fixed period of time. In other embodiments, administration occurs until a particular therapeutic benchmark is reached (e.g., BMD increase is about 3% or higher, at bones such as spine, hip and/or femoral neck). Examples of a suitable timeframe for administration include, without limitation, 6 weeks, 12 weeks, 3 months, 24 weeks, 6 months, 48 weeks, 12 months, 18 months, or 24 months. In certain embodiments, osteoanabolic agents are administered once a day, twice a day, three times a day, or more than three times a day. In other embodiments, administration may occur once every 2 days, once every 3 days, once every 4 days, once per week, or once per month. In certain embodiments, osteoanabolic agents are administered once a day for 18 months.

In certain embodiments, an anti-resorptive agent may be administered to a subject who has received an osteoanabolic agent for an extended period of time. Following the treatment with an osteoanabolic agent, the anti-resorptive agent is administered to the subject for a fixed period of time, such as 6 weeks, 12 weeks, 3 months, 24 weeks, 6 months, 48 weeks, 12 months, 18 months, and 24 months. In certain embodiments, the anti-resorptive agent is administered once a day, twice a day, three times a day, or more than three times a day. In other embodiments, administration may occur once every 2 days, once every 3 days, once every 4 days, once per week, once per month, or once per year. In certain embodiments, the anti-resorptive agent is administered once a day for 6 months, 9 moths or 12 months. In certain embodiments, administration of osteoanabolic agents and the anti-resorptive agent may overlap for some period of time, i.e., administration of the anti-resorptive agent may commence while the subject is still receiving an osteoanabolic agent.

As disclosed herein, overweight subjects treated with osteoanabolic agents exhibit a significant reduction in fractures as compared to the subjects without treatment or subjects treated with a placebo. In certain embodiments, overweight subjects treated with osteoanabolic agents may exhibit a reduction in fractures of at least about 10%, at least about 20%, at least about 30%, at least about 40%, at least about 50%, at least about 60%, at least about 70%, at least about 80%, at least about 90%, or about 100% as compared to untreated subjects or subjects treated with a placebo.

In certain embodiments, the methods provided herein reduce the wrist fracture risk of overweight subjects treated with osteoanabolic agents by about 40% to about 70%, about 50% to about 65%, about 55% to about 60%, or at least about 58% when compared to untreated subjects or subjects treated with placebo. In certain embodiments, the wrist fracture risk for subjects treated with osteoanabolic agents is reduced by about 40% to about 80%, about 50% to about 75%, about 60% to about 75%, about 65% to about 75%, about 70% to about 75%, or at least about 72% compared to untreated subjects or subjects treated with placebo.

In certain embodiments, the methods provided herein reduce the major osteoporotic fracture risk of overweight subjects treated with osteoanabolic agents by about 30% to about 80%, about 40% to about 80%, about 50% to about 75%, about 60% to about 75%, about 65% to about 75%, about 70% to about 75%, about 58%, or at least about 71%, compared to untreated subjects or subjects treated with placebo. In certain embodiments, the major osteoporotic fracture risk for overweight subjects treated with osteoanabolic agents is reduced by about 40% to about 70%, about 50% to about 65%, about 55% to about 60%, or at least about 57% compared to untreated subjects or subjects treated with placebo.

In certain embodiments, the methods provided herein reduce the clinical fracture risk of overweight subjects treated with osteoanabolic agents by about 30% to about 70%, about 35% to about 65%, about 40% to about 60%, about 40 to about 50%, or at least about 45% when compared to untreated subjects or subjects treated with placebo. In certain embodiments, the clinical fracture risk of overweight subjects treated with osteoanabolic agents is reduced by about 15% to about 40%, about 20% to about 35%, about 20% to about 30%, about 20% to about 25%, or at least about 23% compared to untreated subjects or subjects treated with placebo.

In certain embodiments, the methods provided herein reduce the new vertebral fracture risk of overweight subjects treated with osteoanabolic agents by about 50% to about 95%, about 60% to about 95%, about 70% to about 90%, about 80 to about 88%, at least about 87%, or at least about 86% when compared to untreated subjects or subjects treated with placebo. In certain embodiments, subjects treated with osteoanabolic agents exhibit a vertebral fracture risk that is reduced by about 15% to about 45%, about 20% to about 40%, about 25% to about 35%, or at least about 30% versus untreated subjects or subjects treated with placebo.

In certain embodiments, the methods provided herein reduce the non-vertebral fracture risk of overweight subjects treated with osteoanabolic agents by about 30% to about 70%, about 35% to about 65%, about 40% to about 60%, about 40 to about 50%, about 51%, or at least about 45% when compared to untreated subjects or subjects treated with placebo. In certain embodiments, the non-vertebral fracture risk of overweight subjects treated with osteoanabolic agents is reduced by about 15% to about 40%, about 20% to about 35%, about 20% to about 30%, about 20% to about 25%, or at least about 24% compared to untreated subjects or subjects treated with placebo.

In certain embodiments, the methods provided herein result in a significant increase in BMD in the lumbar spine, femoral neck, and total hip of overweight subjects. In certain embodiments, the methods disclosed herein result in a significant BMD increase in lumbar spine, femoral neck, and total hip of overweight subjects within the first year after the first administration of PTHrP or analogues thereof (e.g., abaloparatide) compared to subjects treated with teriparatide. In certain embodiments, the methods disclosed herein result in a significant BMD increase in femoral neck and total hip of overweight subjects compared to subjects treated with teriparatide. In certain embodiments, BMD at the lumbar spine for overweight subjects treated with PTHrP or analogues thereof (e.g., abaloparatide) may increase by at least about 2.9%, at least about 3%, at least about 5.2%, at least about 6%, at least about 6.7%, at least about 12.8%, about 2% to about 8%, about 6% to about 8%, about 2% to about 7%, about 6% to about 7%, about 5.8% to about 7%, about 2% to about 15%, about 6% to about 15%, about 2% to about 14%, about 6% to about 14%, about 2% to about 13%, about 6% to about 13%, about 2% to about 12.8%, about 6% to about 12.8%, or about 5.8% to about 12.8%; BMD at the femoral neck for subjects treated with PTHrP or analogues thereof (e.g., abaloparatide) may increase by at least about 2.2%, at least about 2.7%, at least about 3%, at least about 3.1%, at least about 4.5%, at least about 5%, at least about 6%, about 1.5% to about 4%, about 2% to about 4%, about 2.5% to about 4%, about 2% to about 3.5%, about 1.5% to about 6%, about 2% to about 6%, about 2.5% to about 6%, about 1.5% to about 5%, about 2% to about 5%, about 2.5% to about 5%, about 1.5% to about 4.5%, about 2% to about 4.5%, or about 2.5% to about 4.5%; and BMD for the total hip of subjects treated with PTHrP or analogues thereof (e.g., abaloparatide) may increase by at least about 1.4%, at least about 2.0%, at least about 2.6%, at least about 3%, at least about 3.5%, at least about 4%, at least about 4.5%, at least about 5%, at least about 5.5%, at least about 6%, at least about 7%, about 0.6% to about 3%, about 1% to about 3%, about 1.5% to about 3%, about 0.6% to about 3.5%, about 1% to about 3.5%, about 1.5% to about 3.5%, about 0.6% to about 4%, about 1% to about 4%, about 1.5% to about 4%, about 2% to about 4%, about 0.6% to about 4.5%, about 1% to about 4.5%, about 1.5% to about 4.5%, about 2% to about 4.5%, about 0.6% to about 5%, about 1% to about 5%, about 1.5% to about 5%, about 2.0% to about 5%, about 0.6% to about 5.5%, about 1% to about 5.5%, about 1.5% to about 5.5%, about 2% to about 5.5%, about 0.6% to about 6%, about 1% to about 6%, about 1.5% to about 6%, about 2% to about 6%, about 0.6% to about 6.5%, about 1% to about 6.5%, about 1.5% to about 6.5%, about 2.0% to about 6.5%, about 0.6% to about 7%, about 1% to about 7%, about 1.5% to about 7%, or about 2% to about 7%.

In certain embodiments, overweight subjects are administered PTHrP or analogues thereof (e.g., abaloparatide) at a daily dose of 20 µg, 40 µg, or 80 µg for 24 weeks. In certain embodiments, this administration results in a significant increase in BMD in the lumbar spine, femoral neck, and total hip. In certain embodiments, BMD at the lumbar spine for subjects treated with PTHrP or analogues thereof (e.g., abaloparatide) may increase by at least about 2.9%, at least about 3%, at least about 5.2%, at least about 6%, about 6.7%, at least about 2% to about 8%, at least about 6% to about 8%, at least about 6% to about 7%, or about 5.8% to about 7%; BMD at the femoral neck for subjects treated with PTHrP or analogues thereof (e.g., abaloparatide) may increase by at least about 2.2%, at least about 2.7%, at least about 3.1%, about 2% to about 4%, about 1.5% to about 4%, about 2.5% to about 4%, or about 2% to about 3.5%; and BMD for the total hip of subjects treated with PTHrP or analogues thereof (e.g., abaloparatide) may increase by at least about 1.4%, at least about 2.0%, at least about 2.6%, about 1% to about 3%, about 0.6% to about 3.5%, about 1% to about 3.5%, or about 1.5% to about 3%.

In certain embodiments, subjects are administered with PTHrP or analogues thereof (e.g., abaloparatide) at a daily dose of 20 µg, 40 µg, or 80 µg for 18 months and then administered with alendronate for 6 months with a dosage of 10 mg/day or 70 mg/week (e.g., oral), 5 mg/day or 35 mg/week (e.g., oral), 15 mg/day or 105 mg/week (e.g., oral), 20 mg/day or 140 mg/week (e.g., oral), about 5 to about 20 mg/day or about 35 to about 140 mg/week (e.g., oral), about 5 to about 15 mg/day or about 35 to about 105 mg/week (e.g., oral), about 5 to about 10 mg/day or about 35 to about 70 mg/week (e.g., oral), or about 10 to about 20 mg/day or about 70 to about 140 mg/week (e.g., oral). In certain embodiments, this results in a significant increase in BMD in the lumbar spine, femoral neck, and total hip. In certain embodiments, BMD at the lumbar spine for subjects treated with PTHrP or analogues thereof (e.g., abaloparatide) may increase by at least about 2.9%, at least about 3%, at least about 5.2%, at least about 6%, at least about 6.7%, at least about 12.8%, about 2% to about 8%, about 6% to about 8%, about 2% to about 7%, about 6% to about 7%, about 5.8% to about 7%, about 2% to about 15%, about 6% to about 15%, about 2% to about 14%, about 6% to about 14%, about 2% to about 13%, about 6% to about 13%, about 2% to about 12.8%, about 6% to about 12.8%, or about 5.8% to about 12.8%; BMD at the femoral neck for subjects treated with PTHrP or analogues thereof (e.g., abaloparatide) may increase by at least about 2.2%, at least about 2.7%, at least about 3%, at least about 3.1%, at least about 4.5%, at least about 5%, at least about 6%, about 1.5% to about 4%, about 2% to about 4%, about 2.5% to about 4%, about 2% to about 3.5%, about 1.5% to about 6%, about 2% to about 6%, about 2.5% to about 6%, about 1.5% to about 5%, about 2% to about 5%, about 2.5% to about 5%, about 1.5% to about 4.5%, about 2% to about 4.5%, or about 2.5% to about 4.5%; and BMD for the total hip of subjects treated with PTHrP or analogues thereof (e.g., abaloparatide) may increase by at least about 1.4%, at least about 2.0%, at least about 2.6%, at least about 3%, at least about 3.5%, at least about 4%, at least about 4.5%, at least about 5%, at least about 5.5%, at least about 6%, at least about 7%, about 0.6% to about 3%, about 1% to about 3%, about 1.5% to about 3%, about 0.6% to about 3.5%, about 1% to about 3.5%, about 1.5% to about 3.5%, about 0.6% to about 4%, about 1% to about 4%, about 1.5% to about 4%, about 2% to about 4%, about 0.6% to about 4.5%, about 1% to about 4.5%, about 1.5% to about 4.5%, about 2% to about 4.5%, about 0.6% to about 5%, about 1% to about 5%, about 1.5% to about 5%, about 2.0% to about 5%, about 0.6% to about 5.5%, about 1% to about 5.5%, about 1.5% to about 5.5%, about 2% to about 5.5%, about 0.6% to about 6%, about 1% to about 6%, about 1.5% to about 6%, about 2% to about 6%, about 0.6% to about 6.5%, about 1% to about 6.5%, about 1.5% to about 6.5%, about 2.0% to about 6.5%, about 0.6% to about 7%, about 1% to about 7%, about 1.5% to about 7%, or about 2% to about 7%.

In certain embodiments, subjects are treated with PTHrP or analogues thereof (e.g., abaloparatide) at a daily dose of 20 µg, 40 µg, or 80 µg for 12 weeks to 24 weeks. This administration regimen of abaloparatide has been shown herein to significantly increase TBS in treated subjects, suggesting improved trabecular microarchitecture. In certain embodiments, TBS for subjects treated with PTHrP or analogues thereof (e.g., abaloparatide) for 12 weeks increases by at least about 1.2%, at least about 1.7%, at least about 1.9%, about 1% to about 2.5%, about 1% to about 2%, about 1.6% to about 2.5%, about 1.7% to about 2.5%, about 1.6% to about 2%, or about 1.7% to about 2%. In certain embodiments, TBS for subjects treated with PTHrP or analogues thereof (e.g., abaloparatide) for 24 weeks increases by at least about 2.4%, at least about 2.7%, at least about 3.6%, about 2% to about 4.5%, about 2% to about 4%, about 2.7% to about 4.5%, about 2.7% to about 4%, about 3% to about 4.5%, or about 3% to about 4%.

In certain embodiments of the methods disclosed herein, osteoanabolic agents are administered in combination with one or more additional osteoporosis therapies, including for example an alendronate therapy. In these embodiments, the additional osteoporosis therapy may be administered before, during, or after the treatment with osteoanabolic agents. Osteoanabolic agents and the additional osteoporosis therapy may be administered separately or as part of the same composition. Administration of the two agents may occur at or around the same time, e.g., simultaneously, or the two agents may be administered at different times.

In certain embodiments, osteoanabolic agents and/or the additional osteoporosis therapy are administered in a pharmaceutical composition as the active ingredient(s). Such pharmaceutical composition may further comprise a pharmaceutically acceptable carrier. A "pharmaceutically acceptable carrier" as used herein refers to a pharmaceutically acceptable material, composition, or vehicle that is involved in carrying or transporting a compound or molecule of interest from one tissue, organ, or portion of the body to another tissue, organ, or portion of the body. A pharmaceutically acceptable carrier may comprise a variety of components, including but not limited to a liquid or solid filler, diluent, excipient, solvent, buffer, encapsulating material, surfactant, stabilizing agent, binder, or pigment, or some combination thereof. Each component of the carrier must be "pharmaceutically acceptable" in that it must be compatible with the other ingredients of the composition and must be suitable for contact with any tissue, organ, or portion of the body that it may encounter, meaning that it must not carry a risk of toxicity, irritation, allergic response, immunogenicity, or any other complication that excessively outweighs its therapeutic benefits.

Examples of pharmaceutically acceptable carriers that may be used in conjunction with the compositions provided herein include, but are not limited to, (1) sugars, such as lactose, glucose, sucrose, or mannitol; (2) starches, such as corn starch and potato starch; (3) cellulose and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols such as propylene glycol; (11) polyols such as glycerin, sorbitol, mannitol and polyethylene glycol; (12) esters, such as ethyl oleate and ethyl laurate; (13) disintegrating agents such as agar or calcium carbonate; (14) buffering or pH adjusting agents such as magnesium hydroxide, aluminum hydroxide, sodium chloride, sodium lactate, calcium chloride, and phosphate buffer solutions; (15) alginic acid; (16) pyrogen-free water; (17) isotonic saline; (18) Ringer's solution; (19) alcohols such as ethyl alcohol and propane alcohol; (20) paraffin; (21) lubricants, such as talc, calcium stearate, magnesium stearate, solid polyethylene glycol, or sodium lauryl sulfate; (22) coloring agents or pigments; (23) glidants such as colloidal silicon dioxide, talc, and starch or tri-basic calcium phosphate; (24) other non-toxic compatible substances employed in pharmaceutical compositions such as acetone; and (25) combinations thereof.

The term "about" as used herein means within 10% of a stated value or range of values.

One of ordinary skill in the art will recognize that the various embodiments described herein can be combined. For example, steps from the various methods of treatment disclosed herein may be combined in order to achieve a satisfactory or improved level of treatment.

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention. It will be understood that many variations can be made in the procedures herein described while still remaining within the bounds of the present invention. It is the intention of the inventors that such variations are included within the scope of the invention.

### Examples

### Trial Design:

The ACTIVE pivotal Phase 3 fracture prevention trial for the PTHrP analogue abaloparatide, Study BA058-05-003 (see ClinicalTrials.gov), was a randomized, double-blind, placebo-controlled trial in postmenopausal osteoporotic women randomized to receive daily doses of one of the following for 18 months: 80 micrograms (µg) of abaloparatide; a matching placebo; or the approved daily dose of 20 µg of teriparatide. Treatment with abaloparatide at a daily dose of 80 µg or placebo remained blinded to all parties throughout the study. Teriparatide used was a proprietary prefilled drug and device combination that could not be repackaged. Therefore, its identity could not be blinded to treating physicians and patients once use began. Study medication was self-administered daily by subcutaneous injection for a maximum of 18 months. All enrolled patients also received calcium and vitamin D supplementation from the time of enrollment until the end of the treatment period. It was recommended to patients that they also continue these supplements through the one-month follow-up period.

The trial completed enrollment in March 2013 with 2,463 patients at 28 medical centers in 10 countries in the United States, Europe, Latin America, and Asia. In a post hoc analysis, a subgroup of overweight patients, patients with high BMI (≥ 27 kg/m², ≥ 24 kg/m₂ for Asian patients) was evaluated. The baseline characteristics of this sub-group of patients are detailed in Table 1 below.

**Table 1: Baseline Characteristics of the Selected Patients for post hoc ACTIVE Studies**

| | | Abaloparatide (N=262) | Placebo (N=280) | Teriparatide (N=281 |
|---|---|---|---|---|
| Mean age, years (SD) | | 69.4 (6.1) | 68.8 (6.5) | 69.2 (6.7) |
| Mean weight, kg (SD) | | 69.2 (9.2) | 69.8 (8.3) | 69.9 (8.6) |
| Mean BMI, kg/m² (SD) | | 28.9 (2.2) | 29.1 (2.1) | 29.1 (2.1) |
| Mean BMD T-Score (SD) | | | | |
| | Total hip | -1.66 (0.64) | -1.59 (0.75) | -1.57 (0.72) |
| | Femoral neck | -2.06 (0.58) | -1.97 (0.65) | -1.93 (0.67) |
| | Lumbar Spine | -2.65 (0.90) | -2.70 (0.83) | -2.58 (0.94) |
| Prevalent Vertebral Fracture, N (%) | | 56 (21.4) | 63 (22.5) | 78 (27.8) |
| ≥1 prior nonvertebral fracture within 5 years prior to randomization, N (%) | | 85 (32.4) | 101 (36.1) | 88 (31.3) |
| No prior fractures | | 88 (33.6) | 91 (32.5) | 99 (35.2) |

The study enrolled otherwise healthy ambulatory women aged 49 to 86 (inclusive) who had been postmenopausal for at least five years, met the study entry criteria, and had provided written informed consent. The women enrolled in the study had a BMD T-score ≤ - 2.5 at the lumbar spine or hip (femoral neck) by dual-energy X-ray absorptiometry (DXA), and radiological evidence of two or more mild or one or more moderate lumbar or thoracic vertebral fractures, or history of low trauma forearm, humerus, sacral, pelvic, hip, femoral or tibial fracture within the past five years. Postmenopausal women older than 65 who met the above fracture criteria but had a T-score of <-2.0 could also be enrolled. Women at age 65 or older who did not meet the fracture criteria could also be enrolled if their T-score was <-3.0. All patients were to be in good general health as determined by medical history, physical examination (including vital signs), and clinical laboratory testing. This study population contained a patient population reflective of the type of severe osteoporosis patients that specialists would be expected to treat in their practices.

The study endpoints included BMD, new vertebral fractures, nonvertebral fractures (fractures excluding those of the spine, sternum, patella, digits, skull, and face and those with high trauma), any clinical fractures (all fractures causing a patient to seek medical care), and major osteoporotic fractures (fractures of the upper arm, wrist, hip, or clinical spine). New vertebral fracture compared using Fisher exact test (modified intent-to-treat [mITT] population; participants with pretreatment/postbaseline spine x-rays). Time to first incident nonvertebral, clinical, major osteoporotic, and wrist fracture assessed using Kaplan-Meier (KM) method; log-rank test (intent-to-treat [ITT] population). Change from baseline BMD evaluated for total hip, femoral neck, and lumbar spine (missing data points imputed by last observation carried forward; ITT population).

Safety evaluations performed in the ACTIVE trial included physical examinations, vital signs, 12-lead electrocardiograms, or ECGs, clinical laboratory tests and monitoring, and recording of adverse events. Specific safety assessments included pre-dose and post-dose (four hours) determination of serum calcium, determination of creatinine clearance, post-dose ECG assessments at selected visits, and assessments of postural hypotension (60 minutes post-dose) at selected clinic visits.

### Results

### Reduction in Incidence of New Vertebral Fractures

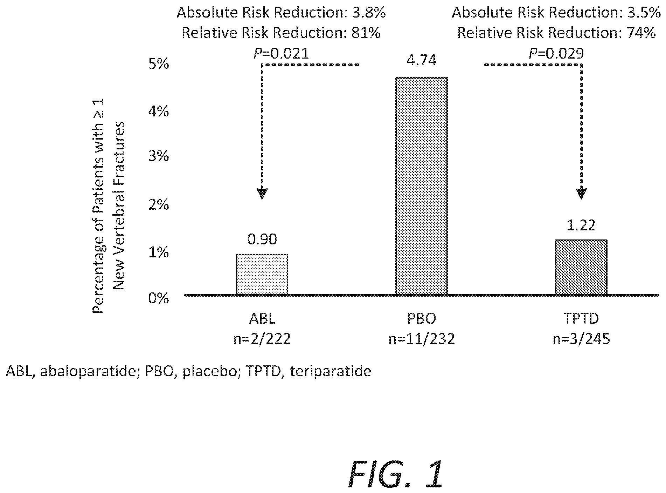

FIG. 1 is a bar graph showing that osteoanabolic agents (both abaloparatide and teriparatide) were associated with a significant reduction in new vertebral fractures, vs. placebo, in an overweight population (i.e., a population having a BMI ≥ 25). Abaloparatide shows a relative risk reduction of 81% versus placebo. Teriparatide shows a relative risk reduction of 74% for teriparatide versus placebo.

### Kaplan-Meir Estimated Cumulative Incidence of Non-Vertebral and Major Osteoporotic Fractures

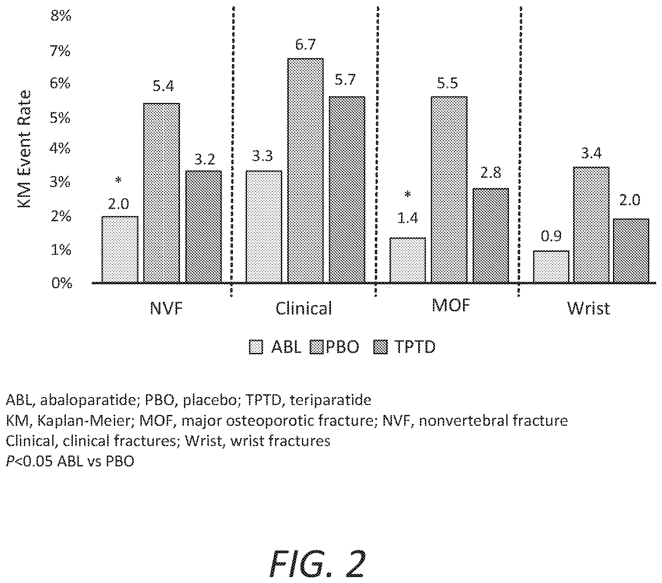

FIG. 2 is a bar graph showing the effect of osteoanabolic agents (both abaloparatide and teriparatide) on non-vertebral fractures (NVF), clinical fractures (Clinical), major osteoporotic fracture (MOF), and wrist fractures (Wrist) in an overweight population (i.e., a population having a BMI ≥ 25). Significant reduction in Kaplan-Meier estimated cumulative incidence of NVF and MOF were observed with osteoanabolic agents versus placebo, as shown in FIG. 2. Abaloparatide performs significantly better than teriparatide in all categories. Fracture reduction trends in overweight patients were consistent with those of the overall ACTIVE population.

### BMD Percent Change from Baseline

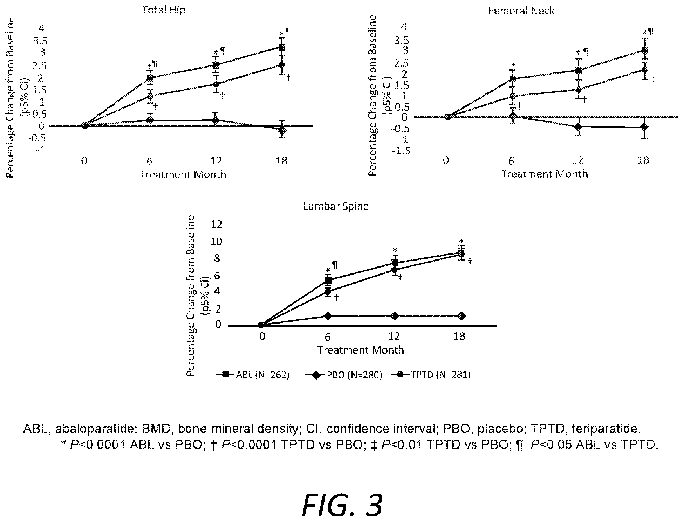

FIG. 3 is a graph depicting changes in BMD at the spine and at non-vertebral sites (total hip and femoral neck) in overweight patients for placebo (diamond), patients treated with abaloparatide (square), and patients treated with teriparatide (circle) over time. Significant increases in bone mineral density were observed with abaloparatide versus placebo at 6, 12, and 18 months at the total hip, femoral neck, and lumbar spine. At all time points, abaloparatide and teriparatide showed significantly greater BMD increase as compared to placebo. Abaloparatide showed significantly greater BMD increase as compared to teriparatide at 6, 12, and 18 months at total hip. Abaloparatide showed significantly greater BMD increase as compared to teriparatide at 12 and 18 months at the femoral neck. Abaloparatide showed significantly greater BMD increase as compared to teriparatide at 6 months for lumbar spine. Moreover, there was a delay of about 6 months in the teriparatide group comparing to the group treated with abaloparatide to achieve the same level of BMD increase at total hip and femoral neck. Therefore, abaloparatide achieved significant results in rapid BMD response.

### Safety

The ACTIVE trial also evaluated several potential safety measures. The treatment-emergent adverse events (TEAEs) reported by the overweight patients are summarized below in Table 2 for groups treated with placebo, abaloparatide, and teriparatide, respectively.

**Table 2: Treatment-Emergent Adverse Events (TEAE)**

| TEAE, n (%) | | Abaloparatide (n=262) | Placebo (n=280) | Teriparatide (n=281) |
|---|---|---|---|---|
| ≥1 TEAE | | 240 (91.6) | 236 (84.3) | 251 (89.3) |
| Serious TEAEs | | 36 (13.7) | 33 (11.8) | 39 (13.9) |
| Deaths^{a} | | 2 (0.8) | 1 (0.4) | 1 (0.4) |
| Discontinuations | | 15 (5.7) | 15 (5.4) | 21 (7.5) |
| Most Frequently Observed AEs | | | | |
| | Dizziness | 29 (11.1) | 17 (6.1) | 23 (8.2) |
| | Hypercalciuria | 25 (9.5) | 24 (8.6) | 26 (9.3) |
| | Hypertension | 25 (9.5) | 18 (6.4) | 18 (6.4) |
| | Arthralgia | 24 (9.2) | 26 (9.3) | 20 (7.1) |
| | Upper respiratory tract infection | 23 (8.8) | 24 (8.6) | 27 (9.6) |
| | Back pain | 21 (8.0) | 25 (8.9) | 21 (7.5) |
| | Urinary tract infection | 18 (6.9) | 15 (5.4) | 19 (6.8) |
| | Pain in extremity | 18 (6.9) | 18 (6.4) | 10 (3.6) |
| | Influenza | 16 (6.1) | 14 (5.0) | 11 (3.9) |
| | Nausea | 16 (6.1) | 9 (3.2) | 11 (3.9) |
| | Creatine renal clearance increased | 15 (5.7) | 17 (6.1) | 13 (4.6) |
| | Headache | 13 (5.0) | 16 (5.7) | 19 (6.8) |
| | Nasopharyngitis | 13 (5.0) | 25 (8.9) | 15 (5.3) |

| | | | | |
|---|---|---|---|---|
| AE, adverse event. ^{a} Causes of death in the placebo group: bowel cancer; abaloparatide group: sepsis, ischemic heart disease; teriparatide group: general health deterioration. ^{b} Occurring in ≥5% of patients in any group. | | | | |

### Discussion

In conclusion, among postmenopausal women with osteoporosis enrolled in ACTIVE, in a subpopulation with high BMI, abaloparatide resulted in significant risk reduction for vertebral, nonvertebral, and major osteoporotic fracture, and numerical reduction in clinical and wrist fracture. Abaloparatide was also associated with significant improvements in bone mineral density at key skeletal sites versus placebo at all time points and versus teriparatide at certain time points. These results are consistent with those observed in the overall ACTIVE population as were changes in bone markers and reported adverse events; confirmation of these findings is warranted. Results from this study suggest abaloparatide may provide a valuable treatment option for overweight women with postmenopausal osteoporosis at high risk for fracture.
The invention also relates to the following numbered embodiments:
1. A method of reducing the incidence of bone fractures in a subject having a body mass index (BMI) greater than or equal to 25, wherein the subject is losing weight at a rate of at least 1 kg/month, the method comprising administering to the subject a therapeutically effective amount of an osteoanabolic agent.
2. A method of reducing the incidence of bone fractures in a subject after the subject has undergone bariatric surgery comprises administering to the subject a therapeutically effective amount of an osteoanabolic agent.
3. The method of embodiment 2, wherein the subject has a body mass index (BMI) greater than or equal to 25 prior to the bariatric surgery.
4. The method of any one of embodiments 1 to 3, wherein the osteoanabolic agent is abaloparatide.
5. The method of embodiment 4, wherein abaloparatide is administered by subcutaneous injection.
6. The method of any one of embodiments 1 to 5, wherein administration of a therapeutically effective amount of an osteoanabolic agent to the subject maintains or increases the subject's bone mineral density (BMD) during weight loss by the patient.
7. The method of any one of embodiments 1 to 6, further comprising administering to the subject a therapeutically effective amount of an anti-resorptive agent.
8. The method of any one of embodiments 1 to 7, wherein the subject is a woman.
9. The method of embodiment 8, wherein the subject is menopausal.
10. The method of any one of embodiments 1 to 9, wherein the subject has osteopenia.
11. The method of any one of embodiments 1 to 10, wherein the subject has a normal BMD prior to treatment with the osteoanabolic agent.
12. The method of any one of embodiments 1 to 11, wherein the method results in a BMD increase of at least about 3% at one or more sites selected from the group consisting of spine, hip, and wrist.

## Claims

1. An osteoanabolic agent for use in a method of reducing the incidence of bone fractures in a subject having a body mass index (BMI) greater than or equal to 25, wherein the subject is losing weight at a rate of at least 1 kg/month, the method comprising administering to the subject a therapeutically effective amount of the osteoanabolic agent.

2. An osteoanabolic agent for use in a method of reducing the incidence of bone fractures in a subject after the subject has undergone bariatric surgery, the method comprising administering to the subject a therapeutically effective amount of the osteoanabolic agent.

3. The osteoanabolic agent for use according to claim 2, wherein the subject has a body mass index (BMI) greater than or equal to 25 prior to the bariatric surgery.

4. The osteoanabolic agent for use according to any one of claims 1 to 3, wherein the osteoanabolic agent is abaloparatide.

5. The osteoanabolic agent for use according to claim 4, wherein the abaloparatide is administered by subcutaneous injection.

6. The osteoanabolic agent for use according to any one of claims 1 to 5, wherein administration of a therapeutically effective amount of an osteoanabolic agent to the subject maintains or increases the subject's bone mineral density (BMD) during weight loss by the subject.

7. The osteoanabolic agent for use according to any one of claims 1 to 6, further comprising administering to the subject a therapeutically effective amount of an anti-resorptive agent.

8. The osteoanabolic agent for use according to any one of claims 1 to 7, wherein the subject is a woman.

9. The osteoanabolic agent for use according to claim 8, wherein the subject is menopausal or post-menopausal.

10. The osteoanabolic agent for use according to any one of claims 1 to 9, wherein the subject has osteopenia.

11. The osteoanabolic agent for use according to any one of claims 1 to 10, wherein the subject has a normal BMD prior to treatment with the osteoanabolic agent.

12. The osteoanabolic agent for use according to any one of claims 1 to 11, wherein the method results in a BMD increase of at least about 3% at one or more sites selected from the group consisting of spine, hip, and wrist.
